# EUROPEAN PATENT APPLICATION

(11) **EP 4 365 191 A1**
(43) Date of publication of application: **08.05.2024**
(21) Application number: 22833489.2
(22) Date of filing: 21.06.2022
(51) Int. Cl.: C07K 14/33, C12N 9/64, A61K 38/48, A61K 47/18, A61K 47/26, A61K 9/00, A61P 17/00

(54) **RECOMBINANT BOTULINUM TOXIN TYPE A LIGHT CHAIN, RECOMBINANT BOTULINUM TOXIN, AND COMPOSITION, USE, AND METHOD THEREOF**

(30) Priority: 28.06.2021 KR 20210083950
(71) Applicant: Medytox Inc., Chungcheongbuk-do 28126 (KR)
(72) Inventor: RHEE, Chang Hoon, Cheongju-si, Chungcheongbuk-do 28123 (KR); KIM, Kook Han, Hwaseong-si, Gyeonggi-do 18447 (KR); LEE, In Tae, Sejong 30062 (KR); KWAK, Seong Sung, Suwon-si, Gyeonggi-do 16228 (KR); AN, Dong Hyun, Suwon-si, Gyeonggi-do 16543 (KR); LEE, Young Rae, Suwon-si, Gyeonggi-do 16225 (KR); CHOI, Hwa Jung, Goyang-si, Gyeonggi-do 10325 (KR)
(74) Representative: Brevalex
(86) International application number: PCT/KR2022/008798
(87) International publication number: WO 2023/277433

(57) **Abstract**

The present disclosure is to provide a recombinant botulinum toxin type A light chain, a recombinant botulinum toxin, and a composition, use, and method related thereto, which exhibit improved efficacy and half-life to be easier to use for patients and enable treatment that is customized to indications.

## Description

### Technical Field

The present disclosure relates to a recombinant botulinum toxin, and more particularly to a recombinant botulinum toxin type A light chain, a recombinant botulinum toxin, and a composition, use, and method related thereto.

### Background Art

A botulinum toxin (BoNT) is a neurotoxic protein produced by the bacterium *Clostridium botulinum* and related species. The BoNT blocks the release of acetylcholine, a neurotransmitter released from axon terminal at the neuromuscular junction.

The BoNT is synthesized as a 150 kDa double-chain protein consisting of a 100 kDa heavy chain and a 50 kDa light chain linked by disulfide bonds. The heavy chain is further divided into an N-terminal domain (Hn) that helps translocate the light chain into the cell's cytosol, and a C-terminal domain (Hc) that recognizes and binds to cell surface receptors on neurons. Once inside the cell, the light chain specifically proteolytic cleaves a portion of the soluble NSF-attachment protein receptor (SNARE) to inactivate neurotransmitter release.

The BoNT is divided into seven serotypes (BoNT type A, BoNT type B, BoNT type C, BoNT type D, BoNT type E, BoNT type F, and BoNT type G), which are further subdivided into subtypes based on variations in amino acid sequence. Among these, BoNT type A1 has been extensively studied at the molecular level and in preclinical and clinical stages, and is currently widely used in the pharmaceutical industry. In contrast, other BoNT type A subtypes have been less studied and reported on their pharmacological properties due to difficulties in isolating purified toxins. Currently, only a portion of the BoNT type A subtypes have been characterized biochemically, cellularly, and in vivo. According to reported studies, various in vitro and in vivo studies on BoNT type A subtype have revealed characteristics that distinguish it from BoNT type A1 in terms of mechanisms such as potency, intracellular movement, and persistence, etc. Recently, the characteristics of BoNT type A7 and BoNT type A8 at the in vitro level has been reported.

International Publication WO2018/132423 discloses botulinum neurotoxin mixtures including a light chain peptide of botulinum neurotoxin type A subtype 1 and a heavy chain peptide of subtype 2, and pharmaceutical compositions including the mixtures, for improved potency, cell entry kinetics, and duration of action. In addition, International Publication WO2017/214447 discloses botulinum toxin B1 with a recombinant heavy chain sequence to maintain the same level of toxin activity at a lower dose.

However, so far, there has been no attempt to improve the potency of recombinant BoNT type A light chain and recombinant BoNT including it, which is a recombinant subdomain of BoNT type A subtype light chain.

### Disclosure

### Technical Problem

An object of the present disclosure is to provide a recombinant botulinum toxin type A light chain, and a recombinant botulinum toxin including the same.

Another objective of the present disclosure is to provide a composition associated with the recombinant botulinum toxin.

Another objective of the present disclosure is to provide a use for the composition associated with the recombinant botulinum toxin.

Another objective of the present disclosure is to provide a method of improving or treating a disease, the method including administering to a subject a composition associated to the recombinant botulinum toxin.

Another objective of the present disclosure is to provide methods of preparing a recombinant botulinum toxin type A light chain, and a recombinant botulinum toxin including the recombinant botulinum toxin type A light chain, which has increased potency or half-life compared to a wild-type botulinum toxin type A light chain, and a wild-type botulinum toxin including the same.

### Technical Solution

One aspect of the present disclosure provides a recombinant botulinum toxin type A light chain in which a sequence of a second domain of first, second, third, and fourth domains of a botulinum toxin type A light chain other than botulinum toxin type A1 is substituted with a sequence of a second domain of botulinum toxin type A1 or a variant thereof.

Another aspect of the present disclosure provides a recombinant botulinum toxin type A light chain, wherein a sequence of the fourth domain of the first, second, third, and fourth domains of the recombinant botulinum toxin type A light chain is further substituted with a sequence of a fourth domain of botulinum toxin type A1 or a variant thereof.

Another aspect of the present disclosure provides a recombinant botulinum toxin including the recombinant botulinum toxin type A light chain and a botulinum toxin heavy chain.

Another aspect of the present disclosure provides a composition including the recombinant botulinum toxin and a pharmaceutically acceptable excipient or additive.

Another aspect of the present disclosure provides a use of the composition including the recombinant botulinum toxin and the pharmaceutically acceptable excipient or additive, for administration to a subject to ameliorate or treat a disease.

Another aspect of the present disclosure provides a method of preparing a recombinant botulinum toxin type A light chain with an increased potency or half-life compared to a wild-type botulinum toxin type A light chain, the method including substituting a sequence of the second domain of the first, second, third, and fourth domains of the botulinum toxin type A light chain other than botulinum toxin type A1 with a sequence of the second domain of botulinum toxin type A1 or a variant thereof.

Another aspect of the present disclosure provides a method of preparing a recombinant botulinum toxin type A light chain with an increased potency or half-life compared to a wild-type botulinum toxin type A light chain, the method including additionally substituting a sequence of the fourth domain of the first, second, third, and fourth domains of the recombinant botulinum toxin type A light chain with a sequence of a fourth domain of botulinum toxin type A1 or a variant thereof.

Another aspect of the present disclosure provides a method of preparing a recombinant botulinum toxin with an increased potency or half-life compared to wild-type botulinum toxin, wherein the recombinant botulinum toxin includes the recombinant botulinum toxin type A light chain and a botulinum toxin heavy chain.

### Advantageous Effects

According to the present disclosure, provided are a recombinant botulinum toxin type A light chain, a recombinant botulinum toxin, and a composition, use, and method related thereto, which increase the potency and half-life, increase patient convenience, and allow customized treatment for indications.

### Description of Drawings

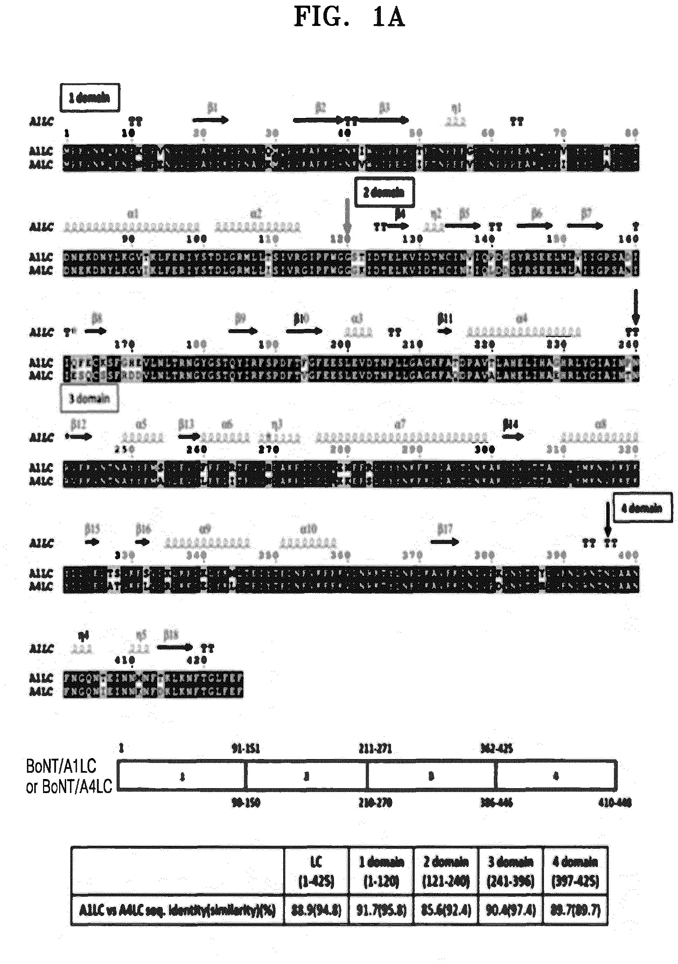

FIG. 1A illustrates sequence comparison (ESPript3.0, Robert and Gouet, 2014) of botulinum toxin type A1 light chain (GenBank: CAL82360.1), and botulinum toxin type A4 light chain (GenBank: ACQ51417.1). The boundaries and starting points of each domain for domain substitutions are indicated by arrows and boxes, and the table below shows the sequence coverage of each domain and the results of the amino acid identity analysis between the two sequences.

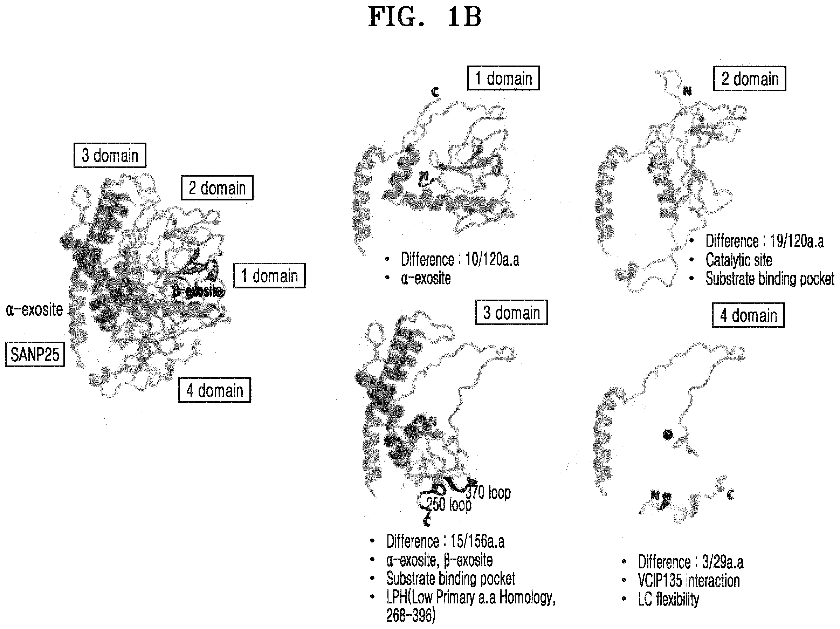

FIG. 1B illustrates structural information about the domains obtained by dividing the botulinum toxin type A1 light chain and the botulinum toxin type A4 light chain, and illustrates the amino acid sequence differences and functional properties for each domain.

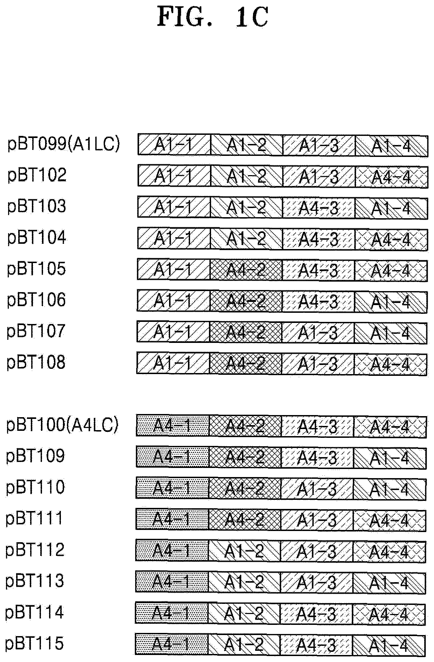

FIG. 1C is a schematic diagram illustrating a combination of a botulinum toxin type A1 light chain and a recombinant botulinum toxin type A light chain in which the domains of the botulinum toxin type A4 light chain have been substituted.

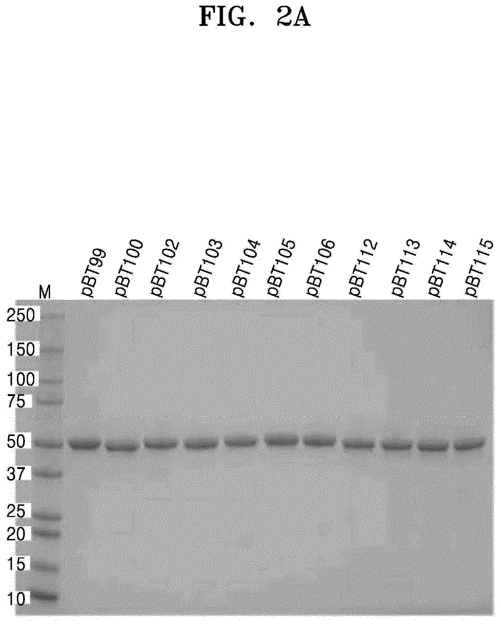

FIG. 2A is an sodium dodecyl-sulfate polyacrylamide gel electrophoresis (SDS-PAGE) analysis of the domain substituted and purified recombinant botulinum toxin type A light chain.

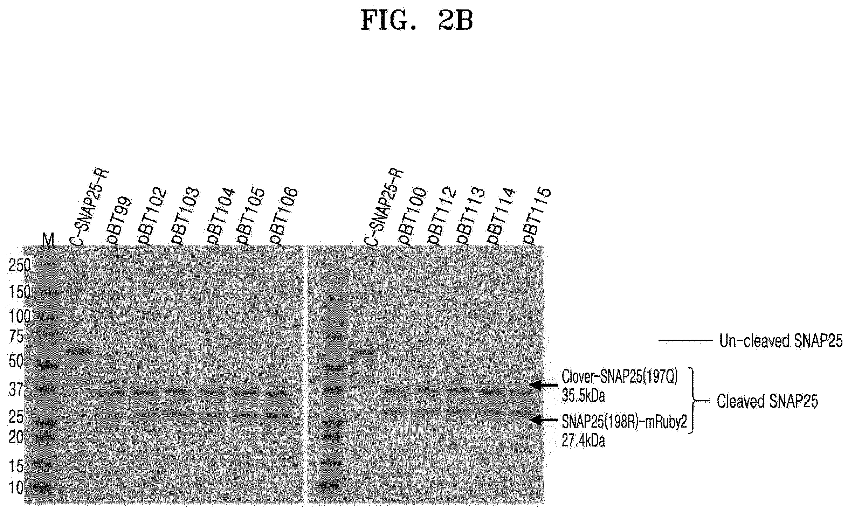

FIG. 2B shows the results of confirming the region where Clover-SNAP25-mRuby2(137-206) was proteolytic cleaved by the domain substituted recombinant botulinum toxin type A light chain. As a result of confirming the presence or absence of proteolytic cleavage of a fluorescent protein-labeled SNAP25(137-206) under the condition of the presence and absence of domain substituted recombinant botulinum toxin type A light chain, it was proteolytic cleaved in the presence of the domain substituted recombinant botulinum toxin type A light chain. Samples were analyzed by SDS-PAGE and Coomassie blue staining.

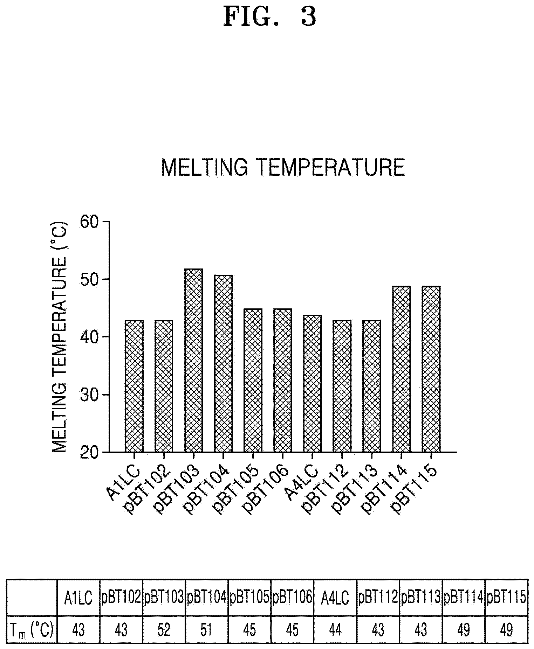

FIG. 3 is a graph illustrating the thermal stability of the domain substituted recombinant botulinum toxin type A light chain. The table below illustrates the melting temperature of each domain substituted recombinant botulinum toxin light chain.

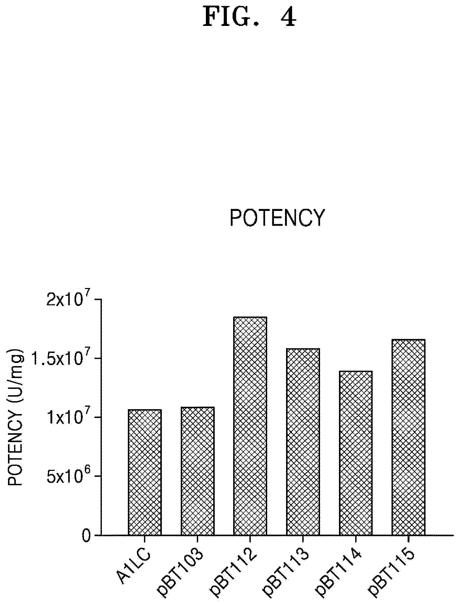

FIG. 4 shows the results of an endopeptidase assay of a domain substituted recombinant botulinum toxin type A light chain. Comparing the potency results of the domain substituted recombinant botulinum toxin type A light chain and the wild-type botulinum toxin type A light chain, pBT103 showed a similar potency to the wild-type, while pBT112 increased by 1.73-fold, pBT113 by 1.49-fold, pBT114 by 1.3-fold, and pBT115 by 1.55-fold.

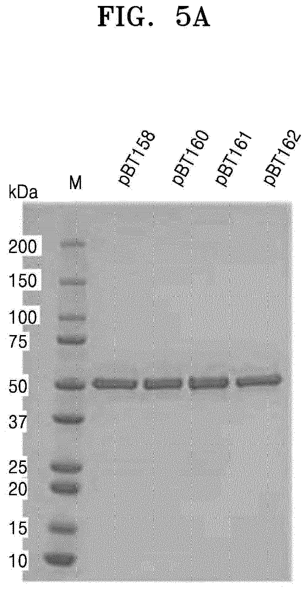

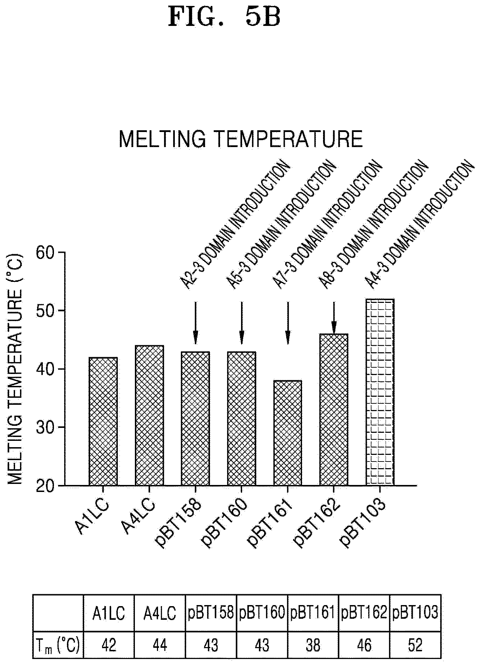

FIG. 5 shows the results of SDS-PAGE analysis (FIG. 5A) and the results of thermal stability analysis (FIG. 5B) of a purified product of a recombinant botulinum toxin type A light chain in which the light chain of botulinum toxin type A was substituted with the third domain of a different botulinum toxin type A subtype. The melting point of each recombinant botulinum toxin type A light chain is shown in the table below. When the third domain of the botulinum toxin type A4 light chain was introduced into the botulinum toxin type A light chain, thermal stability was improved the most.

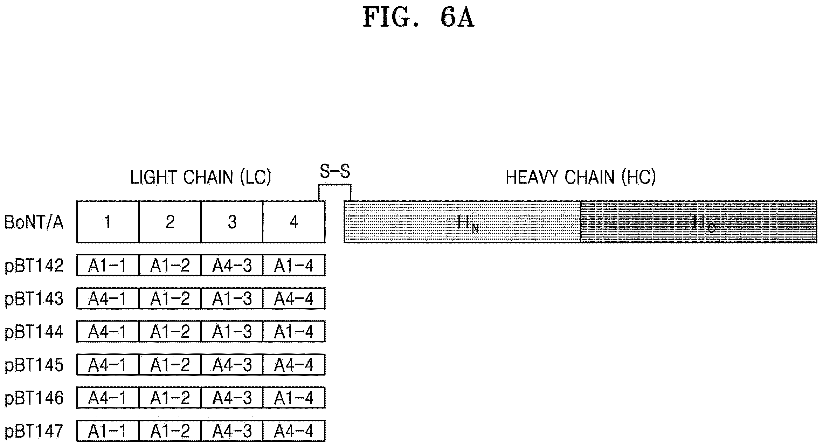

FIG. 6A is a schematic diagram illustrating a composition of a recombinant botulinum toxin including a domain substituted recombinant botulinum toxin type A light chain.

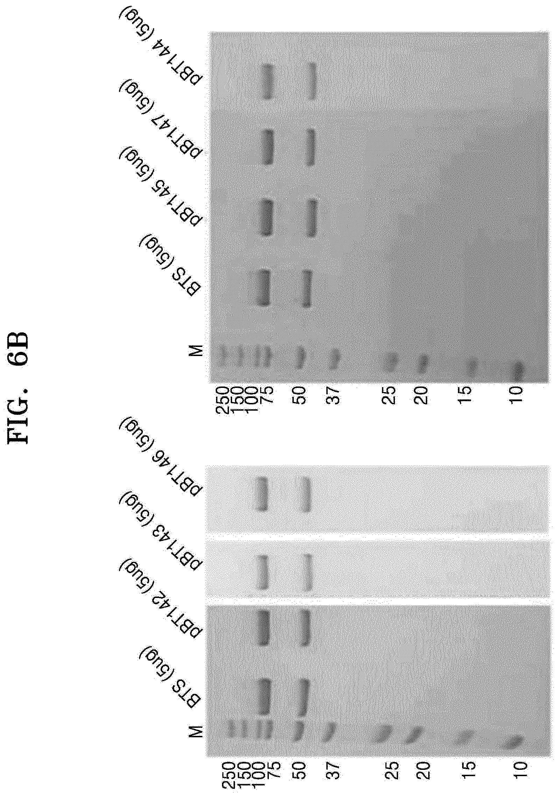

FIG. 6B shows the results of SDS-PAGE analysis under reducing conditions on the purified product of the recombinant botulinum toxin into which the domain substituted recombinant botulinum toxin type A light chain was introduced. The recombinant botulinum toxin was purified into a 150 kd form without impurities and then analyzed by SDS-PAGE under reducing conditions.

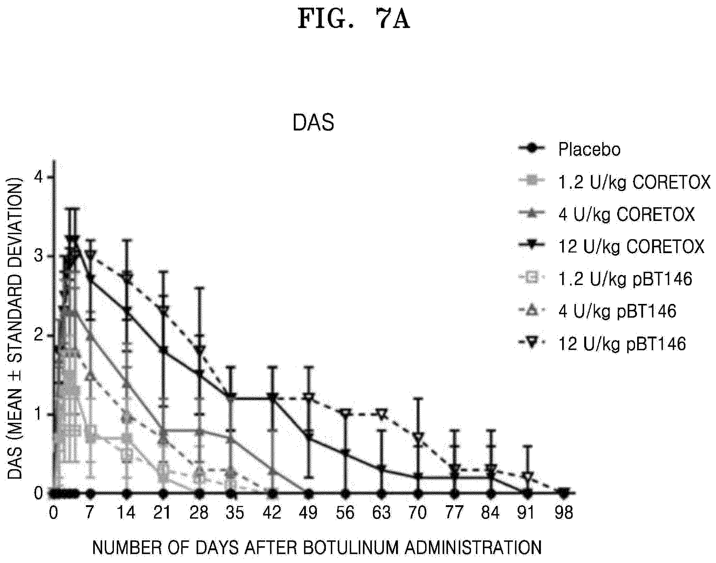

FIG. 7A is a graph illustrating changes in DAS levels over time in rats after administering Coretox (botulinum toxin type A product) and recombinant botulinum toxin (pBT146) to the right gastrocnemius muscle at doses of 1.2, 4, and 12 U/kg, respectively. DAS values on each measurement day were analyzed using the Mann-Whitney test. (*p<0.05)

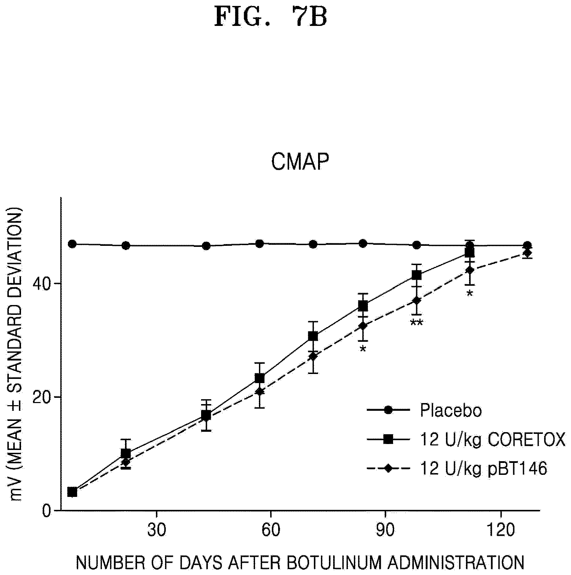

FIG. 7B is a graph illustrating changes in CMAP levels over time in rats after administering Coretox and recombinant botulinum toxin (pBT146) at a dose of 12 U/kg to the right gastrocnemius muscle. The results of analysis using a two-tailed t test for CMAP values of Coretox and recombinant botulinum toxin (pBT146) on each measurement day are shown. (*p<0.05, **p<0.01)

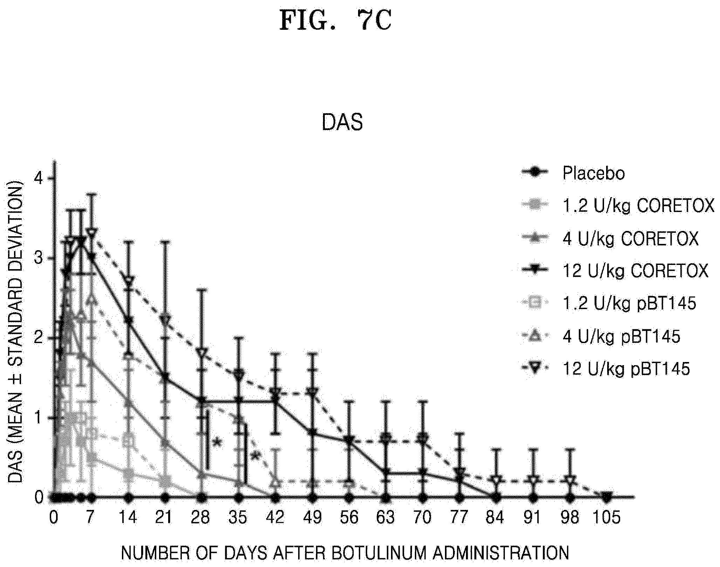

FIG. 7C is a graph illustrating changes in DAS levels over time in rats after administering Coretox and recombinant botulinum toxin (pBT145) to the right gastrocnemius muscle at doses of 1.2, 4, and 12 U/kg, respectively. DAS values on each measurement day were analyzed using the Mann-Whitney test. (*p<0.05, **p<0.01)

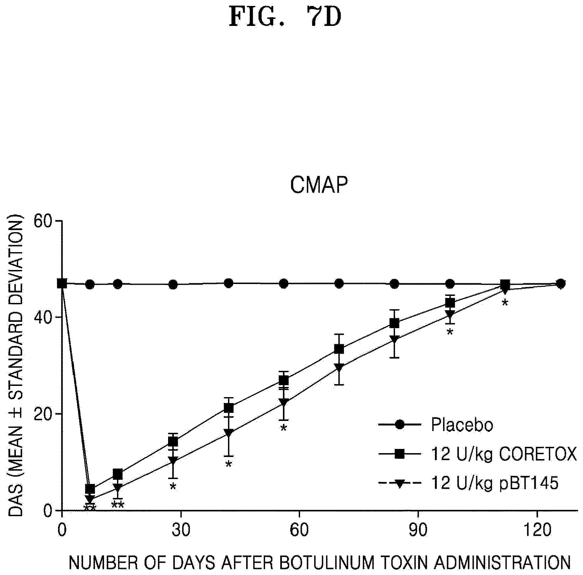

FIG. 7D is a graph illustrating changes in CMAP levels over time in rats after administering Coretox and recombinant botulinum toxin (pBT145) to the right gastrocnemius muscle at a dose of 12 U/kg, respectively. The results of analysis using a two-tailed t test for CMAP values of Coretox and recombinant botulinum toxin (pBT145) on each measurement day are shown. (*p<0.05, **p<0.01)

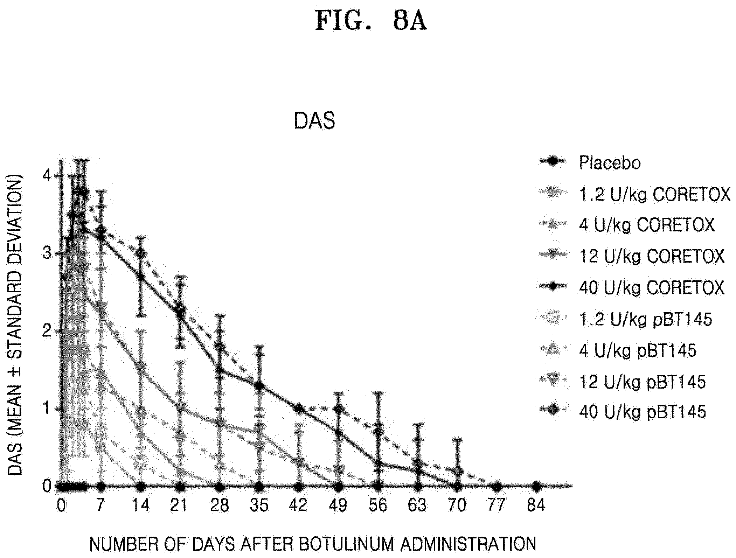

FIG. 8A is a graph illustrating changes in DAS levels over time in mice after administering Coretox (botulinum toxin type A product) and recombinant botulinum toxin (pBT145) to the right gastrocnemius muscle at doses of 1.2, 4, 12, and 40 U/kg, respectively.

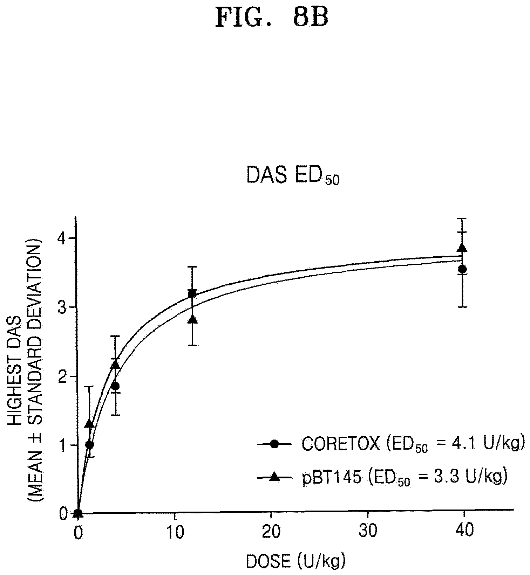

FIG. 8B is a graph illustrating the trend line for the highest DAS score and the calculated DAS ED₅₀ at each dose after administering Coretox and recombinant botulinum toxin (pBT145) to the right gastrocnemius muscle in mice at doses of 1.2, 4, 12, and 40 U/kg, respectively.

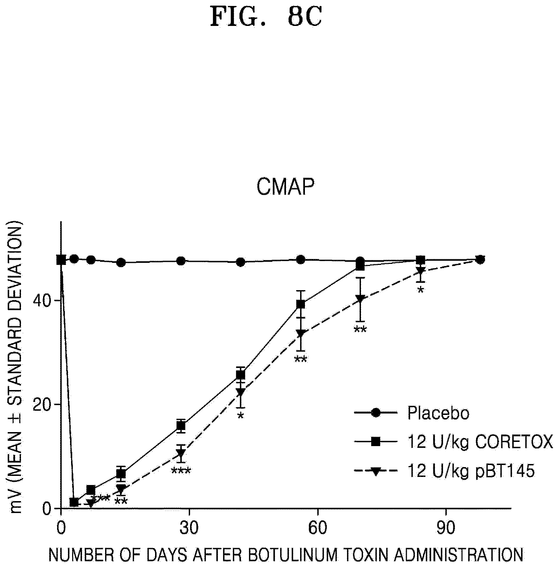

FIG. 8C is a graph illustrating changes in CMAP levels over time in mice after administering Coretox and recombinant botulinum toxin (pBT145) to the right gastrocnemius muscle at a dose of 12 U/kg, respectively. The CMAP values of Coretox (botulinum toxin Type A product) and recombinant botulinum toxin (pBT142, and pBT147) on each measurement day were analyzed using a two-tailed t test. (*p<0.05, **p<0.01, ***p<0.001)

### Best Mode

Hereinafter, the present disclosure will be described in more detail using specific examples, but this is only intended to aid understanding of the present disclosure and is not intended to limit the scope of the present disclosure in any way.

### Example 1: Domain substitution in botulinum toxin type A light chain

In order to determine which domain affects protein stability in the botulinum toxin type A1 light chain and botulinum toxin type A4 light chain, four domains of the botulinum toxin type A1 light chain and botulinum toxin type A4 light chain genes were substituted. Using the in-fusion cloning method, a total of 16 domain substituted light chain genes, including a domain substituted gene and a wild-type, were cloned into the *Escherichia coli* (*E. Coli*) expression vector pET28a, and were named pBT1 02 to pBT1 08 and pBT1 09 to pBT115, respectively (FIG. 1C). Afterwards, the domain substitution was confirmed by analyzing the recombinant botulinum toxin A light chain gene sequence.

### Example 2: Expression and purification of domain substituted botulinum toxin type A light chain

Using the recombinant botulinum toxin type A light chain gene prepared in Example 1, 14 types of recombinant botulinum toxin type A light chains were prepared. The prepared recombinant expression vector was introduced into BL21 (DE3) cells, a type of *E. Coli*, to induce expression of a domain substituted recombinant botulinum toxin type A light chain. Expression was induced with Isopropyl β-D-1-thiogalactopyranoside (IPTG) and used to a final concentration of 0.5 mM. That is, after adding 0.5 ml of 1 M IPTG to 1 L of liquid medium, BL21(DE3) cells cultured for about 3 hours at 37 °C and 18 hours at 18 °C were centrifuged at 4,000 rpm for 10 minutes to precipitate the cells. The supernatant medium was removed, the precipitated cells were resuspended with A buffer (20 mM Tris-HCl, 300 mM NaCl, 10 mM Imidazole, 2 mM B-Me, pH 7.9), and the cells were disrupted on ice using an ultrasonic disperser at 2 pulses/1 pause and power 35 % for 20 minutes. After centrifuging the disrupted cells for 1 hour at 4°C at 14,000 rpm, the recombinant botulinum toxin type A light chain was separated from the supernatant from which cell debris was removed through the combination of His tag and Ni-NTA resin using a Ni-NTA column. The amino acid sequences of the recombinant botulinum toxin type A light chain were designated as pBT102 (SEQ ID NO: 18), pBT103 (SEQ ID NO: 19), pBT104 (SEQ ID NO: 20), pBT105 (SEQ ID NO: 21), pBT106 (SEQ ID NO: 22), and pBT107 (SEQ ID NO: 23), respectively, pBT108 (SEQ ID NO: 24), pBT109 (SEQ ID NO: 25), pBT110 (SEQ ID NO: 26), pBT111 (SEQ ID NO: 27), pBT112 (SEQ ID NO: 28), pBT113 (SEQ ID NO: 29), pBT114 (SEQ ID NO: 30), and pBT115 (SEQ ID NO: 31).

A total of 16 types of proteins, including the recombinant botulinum toxin type A light chain and wild-type botulinum toxin type A1 and A4, were attempted to be expressed, but 5 types were expressed in low amounts or expressed as an inclusion body, and ultimately, 11 types of proteins were expressed in soluble form (FIG. 2A). Afterwards, the 11 purified proteins were reacted with SNAP25 (137-206) substrate labeled with fluorescent protein at the N-terminus and C-terminus, and protease activity was confirmed. 3 µM of fluorescent protein-labeled SNAP25 (137-206) was mixed with 0.2 µM of domain substituted recombinant botulinum toxin light chain, incubated at 37 °C for 2 hours, and loaded on SDS-PAGE. Through Coomassie blue staining, it was confirmed that fluorescent protein-tagged SNAP25(137-206) was proteolytic cleaved by domain substituted recombinant botulinum toxin type A light chain (FIG. 2B).

### Test Example 1: Evaluation of thermal stability of domain substituted botulinum toxin type A light chain

The thermal stability of the domain substituted recombinant botulinum toxin type A light chain was compared. As a stability evaluation method, heat denaturation midpoint temperature (Tm) was evaluated, in general, it is preferable that the heat denaturation midpoint temperature is high. The thermal stability of each domain substituted recombinant botulinum toxin type A light chain was evaluated by protein thermal shift (PTS). Thermal shift assay was performed according to the manufacturer's method using the Protein Thermal Shift Dye Kit (Catalog No. 4461146, Applied Biosystems). Specifically, 10 µg of each recombinant botulinum toxin type A light chain was mixed with the protein thermal shift dye and buffer to 20 µL, and the ROX signal was confirmed by RT-PCR (C1000 thermal cycler equipped with a CFX96 optical reaction module, Bio-Rad) from 20 °C to 95 °C with a temperature increase of 1 °C per 60 seconds. Based on the melting curve, melting temperature (Tm) of each recombinant botulinum toxin light chain was determined. The melting temperature of each recombinant botulinum toxin type A light chain in which the above domain was substituted is shown in FIG. 3.

The results confirmed that the thermal stability of pBT114 (SEQ ID NO: 30) and pBT115 (SEQ ID NO: 31) increased. While the melting temperature of the wild-type botulinum toxin type A1 light chain was 43 °C, the melting temperature of pBT103 and pBT104 increased to 52 °C and 51 °C, respectively, and those of pBT114 and pBT115 increased to 49 °C. Therefore, the thermal stability of pBT103 and pBT104 increased at 8 °C and 9 °C compared to the melting temperature of the wild-type botulinum toxin type A1 light chain used as a control group, and the thermal stability of pBT114 and pBT115 increased at 6 °C. Therefore, it was confirmed that the melting temperature of the recombinant botulinum toxin type A light chain, which was domain substituted to include the second domain of the botulinum toxin type A1 light chain, was commonly increased.

### Test Example 2: Potency assessment of domain substituted botulinum toxin type A light chain

To confirm the in vitro activity of domain-substituted recombinant botulinum toxin type A light chain, an enzyme-linked immunosorbent assay (ELISA)-based endopeptidase assay was performed. First, GST-SNAP25 (137-206) substrate was diluted to a final concentration of 2 µg/mL, and then 100 µL per well was added to a 96 well plate and reacted at 4 °C for 16 hours. 2 % skim milk was prepared by dissolving it in phosphate buffered saline with tween (PBST), and then 200 µL was added to the substrate-coated well and reacted at 25 °C for 1 hour. After the reaction, the wells were washed 3 times with 300 µL of PBST, and then a standard sample (STD (1, 2, 3, 4, 5, 6 U/mL) samples) and a domain substituted recombinant botulinum toxin type A light chain sample (target concentration: 4U/mL) were prepared using Toxin Assay Buffer (reaction buffer containing HEPES, DTT, Zinc sulfate, and Tween-20), 100 µL each was loaded into each well and reacted at 25°C for 2 hours. After reaction, wells were washed 3 times using 300 µL of PBST, primary antibody Rabbit anti-SNAP25 (190-197) pAb was added to 100 µL/well, reacted for 30 minutes, and washed again 3 times as above, then secondary antibody Anti-rabbit IgG HRP conjugate was added and reacted for 30 minutes. TMB (BioFx) solution was added at 100 µL/well and color was developed at 25 °C for 30 minutes. When color development was completed, 50 µL/well of 3M sulfuric acid was added to stop color development, and the absorbance was measured at 450 nm.

After drawing a calibration curve between the concentration of the standard sample and the optical density using the optical density (OD) of the standard sample, the measured potency of the domain substituted recombinant botulinum toxin type A light chain sample was obtained using the generated linear equation. Using the calculated potency and target concentration, the recovery rate of the domain substituted recombinant botulinum toxin type A light chain sample was calculated to confirm the recovery rate compared to the target concentration, and the results are shown in FIG. 4. As shown in FIG. 4, pBT103 (SEQ ID NO: 19) showed similar potency to wild-type botulinum toxin type A1 light chain, while pBT114 (SEQ ID NO: 30) showed a 1.3-fold and pBT115 (SEQ ID NO: 31) a 1.55-fold increase in potency.

### Comparative Example 1: Expression, purification and thermal stability evaluation of recombinant botulinum toxin type A light chain substituted with light chain third domain of different botulinum toxin type A subtype

To determine whether the third domain of A4 in the recombinant botulinum toxin type A light chain is an important factor in the increased thermal stability, the thermal stability was compared after the third domain of the botulinum toxin type A2, A5, A7, and A8 light chain, which are botulinum toxin type A other than botulinum toxin type A4 light chain, was substituted with the third domain of botulinum toxin type A1 light chain.

In order to secure recombinant botulinum toxin type A light chain with the third domain substituted, four types of recombinant botulinum toxin type A light chain were purified according to the purification method in Example 2, and recombinant botulinum toxin type A light chain with substituted third domain of the light chain of each botulinum toxin type A subtype was cloned into pET28a, an *Escherichia coli* (*E. Coli*) expression vector.

The vector substituted with the third domain of botulinum toxin type A2 light chain was called pBT158 (SEQ ID NO: 32) and the vector substituted with the third domain of botulinum toxin type A5 light chain was called pBT160 (SEQ ID NO: 33), the vector substituted with the third domain of botulinum toxin type A7 light chain was named pBT161 (SEQ ID NO: 34), and the vector substituted with the third domain of botulinum toxin type A8 light chain was named pBT162 (SEQ ID NO: 35). Each recombinant botulinum toxin type A light chain was purified in the same manner as in Example 2 (FIG. 5A). The recombinant botulinum toxin light chain protein in which the third domain of the botulinum toxin type A3 light chain was substituted was not expressed, and the third domain of the botulinum toxin type A6 light chain had the same sequence as the third domain of A1, so it was excluded from the test.

As a result of substituting the third domain of the botulinum toxin type A1 light chain with the third domain of the botulinum toxin type A2 light chain, the third domain of the botulinum toxin type A5 light chain, the third domain of the botulinum toxin type A7 light chain, or the third domain of the botulinum toxin type A8 light chain as above, the recombinant botulinum toxin type A light chain, in which the third domain of the botulinum toxin type A1 light chain was substituted with the third domain of botulinum toxin type A4, had the highest melting temperature, that is, the best thermal stability (FIG. 5B).

### Example 3: Preparation and purification of domain substituted recombinant botulinum toxin

To analyze the function of the recombinant botulinum toxin with respect to the domain-substituted recombinant botulinum toxin type A light chain, a recombinant full-length botulinum toxin gene, which includes the recombinant botulinum toxin type A light chain with increased thermal stability and increased activity, was cloned by an infusion method (infusion-HD, Takara) using the pMTL80000 vector system. The cloned products were named pBT142, pBT143, pBT144, pBT145, pBT146, and pBT147, and the composition of each domain is schematically shown in FIG. 6A. The domain substituted recombinant botulinum toxin was used by producing a detoxified hall A-hyper strain in which the toxin gene was inactivated using the ClosTron system.

Detoxified clostridium botulinum strain, including strain in which the toxin gene is inactivated or knocked out, may be used in a method of preparing purified recombinant botulinum toxin or purified recombinant botulinum toxin complex. Methods for producing strains in which the toxin gene is inactivated or knocked out are known in the art, for example using the ClosTron system is described in the literature [Bradshaw et al. 2010, Pellett et al. 2016].

The recombinant botulinum toxin prepared by the ClosTron system was purified into a 150 kDa form that does not include complex components (FIG. 6A). The recombinant botulinum toxins were confirmed to be pBT142 (SEQ ID NO: 36), pBT143 (SEQ ID NO: 37), pBT144 (SEQ ID NO: 38), pBT145 (SEQ ID NO: 39), pBT146 (SEQ ID NO: 40), and pBT147 (SEQ ID NO: 41), respectively. All manufacturing of the above recombinant botulinum toxin was performed in a facility licensed for toxin production in accordance with government regulations.

### Test Example 3: Potency of domain substituted recombinant botulinum toxin in rat

Female 6-week-old Sprague-Dawley (SD) rats were purchased from Orient Bio Co., Ltd., and after acclimatization and quarantine for 1 week, 7-week-old rats were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee.

As a control group, 100 units of Coretox (botulinum toxin type A, Lot No.: NSA19016 or NSA20011, Medytox), a botulinum toxin product, was used. Recombinant botulinum toxin pBT145 was prepared at 304 U/mL and pBT146 at 243 U/mL and used in the experiment. The potency of each recombinant botulinum toxin was tested using a mouse potency test method based on the potency value derived from measurement before the potency test. As a placebo (placebo administration group), a potency test was conducted on recombinant botulinum toxin pBT145 and pBT146, and Coretox, using the same excipient components except for the botulinum toxin component of the recombinant botulinum toxin. The administered dose of each test substance was administered at the same dose based on unit (U: a value representing the biological activity of botulinum toxin, where 1U is the half-lethal dose of mice administered by intraperitoneal injection).

Coretox was diluted using sterilized saline solution (35V3AF3, Daehan Pharmaceutical Industry), and recombinant botulinum toxin pBT145 and pBT146 were diluted using placebo to concentrations of 12, 40, and 120 U/mL, respectively. The day the test substance was administered was set as day 0, after anesthetizing the rats using an injection anesthetic (60 mg/kg ketamine hydrochloride + 10 mg/kg xylazine), each test substance was administered at 0.1 mL/kg to the rat's right gastrocnemius muscle using a Hamilton syringe according to the composition of each group in Table 1.

For potency evaluation, the digit abduction score (DAS) evaluation method, which visually evaluates the degree of muscle paralysis in rats, and the compound muscle action potential (CMAP) test method, which measures the action potential of muscles responding to external electrical stimulation, were used.

**[Table 1]**

| | Group | Number of animals | Administer ed substance | Dose (U/kg) | Administration route | Evaluation indicator |
|---|---|---|---|---|---|---|
| Experi ment 1 | 1 | 6 | Placebo | 0 | Right gastrocnemius muscle | DAS, CMAP |
| | 2 | 6 | Coretox | 1.2 | Right gastrocnemius muscle | DAS |
| | 3 | 6 | Coretox | 4 | Right gastrocnemius muscle | DAS |
| | 4 | 6 | Coretox | 12 | Right gastrocnemius muscle | DAS, CMAP |
| | 5 | 6 | pBT146 | 1.2 | Right gastrocnemius muscle | DAS |
| | 6 | 6 | pBT146 | 4 | Right gastrocnemius muscle | DAS |
| | 7 | 6 | pBT146 | 12 | Right gastrocnemius muscle | DAS, CMAP |
| Experi ment 2 | 1 | 6 | Placebo | 0 | Right gastrocnemius muscle | DAS, CMAP |
| | 2 | 6 | Coretox | 1.2 | Right gastrocnemius muscle | DAS |
| | 3 | 6 | Coretox | 4 | Right gastrocnemius muscle | DAS |
| | 4 | 6 | Coretox | 12 | Right gastrocnemius muscle | DAS, CMAP |
| | 5 | 6 | pBT145 | 1.2 | Right gastrocnemius muscle | DAS |
| | 6 | 6 | pBT145 | 4 | Right gastrocnemius muscle | DAS |
| | 7 | 6 | pBT145 | 12 | Right gastrocnemius muscle | DAS, CMAP |

The DAS value shown in Table 2 indicate the degree of muscle paralysis according to the form of the toe on the administered side in rats, and the CMAP value indicate the degree of inhibition of direct muscle contraction caused by nerve block.

**[Table 2]**

| Score | Criteria |
|---|---|
| 0 | Normal |
| 1 | Loss of abduction in the fifth toe |
| 2 | Three toes stuck together |
| 3 | Four toes stuck together |
| 4 | All toes have loss of abduction and are stuck together |

CMAP was measured in the right gastrocnemius muscle area of each rat using a Nicolet Viking Quest (Viasys Healthcare, Inc.) equipment. After anesthesia with an injection anesthetic, fur was removed from the measurement area and the rat was placed in a prone position. The cathode was placed on the sciatic nerve area on the side of the leg to be measured, and the anode was placed approximately 1 cm away from the area based on the spine. The recording electrode and reference electrode were placed on the gastrocnemius muscle region and achilles tendon region, respectively, and the ground electrode was placed on the rectus femoris region. The level and duration of stimulation were 25 mA to 30 mA, 0.2 ms, and the filter range of the amplifier was set to 2 K to 10 K at 60 Hz. When measured under the relevant conditions, the height from the base of the waveform to the peak was converted into CMAP measurement data. According to the test group composition in Table 1, DAS measurement to compare the potency of pBT146 and Coretox in Experiment 1 was evaluated from day 1 to day 98 after administration at doses of 1.2, 4, and 12 U/kg, and CMAP measurement was measured from day 8 to day 127 after administration in subjects administered at a dose of 12 U/kg. To compare potency between pBT145 and Coretox in Experiment 2, DAS measurement was evaluated from day 1 to day 105 after administration at doses of 1.2, 4, and 12 U/kg, and CMAP measurement was measured from day 0 to day 126 after administration in subjects administered at a dose of 12 U/kg.

Graphpad Prism 7.05 (GraphPad Software Inc., CA, USA) was used for graph presentation, and SPSS software 25.0 (SPSS Inc., IL, USA) and Excel (2013, MS, USA) were used for statistical analysis. The result of the experiment was expressed as mean ± standard deviation. DAS ED₅₀ was calculated using a 3-index logistic model after fixing the lowest score of 0 and the highest score of 4 using Graphpad Prism. Normality was tested using the Kolmogorov-Smirnov test. Non-parametric data were statistically analyzed using the Mann-Whitney test, and for parametric data, a two-tailed t test was conducted, and if the p-value was less than 0.05, it was judged to be statistically significant.

After administering a single dose of Coretox and recombinant botulinum toxin pBT146 at doses of 1.2, 4, and 12 U/kg to the right gastrocnemius muscle of a rat, DAS evaluation results showed that all subjects in the Coretox 1.2 U/kg administration group recovered on day 28. In the group administered with the same dose of pBT146, all subjects recovered on day 42, an increase of 14 days. In addition, in the Coretox 12 U/kg administration group, all subjects recovered on day 91 after administration, but in the group administered with the same dose of pBT146, all subjects recovered on day 98, an increase of 7 days (FIG. 7A). In the CMAP test, the pBT146 12 U/kg administration group showed significantly lower CMAP values compared to the Coretox 12 U/kg administration group on days 84, 98, and 112 after administration, showing strong potency. In the Coretox 12 U/kg administration group recovered the CMAP value on day 112 after administration, and the pBT146 12 U/kg administered group recovered the CMAP value on day 127, an increase of 15 days (FIG. 7B). As mentioned above, it was confirmed that the potency and duration of action of recombinant botulinum toxin pBT146 increased compared to Coretox.

After administering a single dose of Coretox and recombinant botulinum toxin pBT145 at doses of 1.2, 4, and 12 U/kg to the right gastrocnemius muscle of a rat, DASevaluation results showed that the DAS score significantly increased in the pBT145 administration group compared to the Coretox administration group on days 28, 35 after administration at a dose of 4 U/kg. In the Coretox 4 U/kg administration group, all subjects recovered on day 42, and in the group administered with the same dose of pBT145, all subjects recovered on day 63, an increase of 21 days. In the Coretox 12 U/kg administration group, all subjects recovered on day 84 after administration, but in the group administered with the same dose of pBT145, all subjects recovered on day 105, an increase of 21 days (FIG. 7C). In the CMAP test, it was confirmed that the pBT145 12 U/kg administration group showed significantly lower CMAP values compared to the Coretox 12 U/kg administration group at days 7, 14, 28, 42, 56, 98, and 112 after administration, showing strong potency, and in the Coretox 12 U/kg administration group, CMAP recovery was observed on day 112 after administration, and in the pBT145 12 U/kg administration group, CMAP recovery was observed on day 126, 14 days longer than this (Figure 7D). From the above results, it was confirmed that the potency and duration of action of recombinant botulinum toxin pBT145 increased compared to Coretox.

### Test Example 4: Potency of domain substituted recombinant botulinum toxin in mice

Female 5-week-old CD1 (ICR) mice were purchased from Orient Bio Co., Ltd., and after acclimatization and quarantine for 1 week, 6-week-old mice were used in the experiment. The feed was supplied ad libitum with sterilized solid feed for laboratory animals (R40-10, SAFE, France), and drinking water was supplied ad libitum with autoclaved tap water. During the acclimation, quarantine, and experimental period, mice were housed under specific-pathogen-free conditions set at a temperature of 23 ± 3 °C, relative humidity of 55 ± 15 %, illumination time of 12 hours (8:00 a.m. to 8:00 p.m.), ventilation frequency of 15 times/hour, and illumination intensity of 150 to 300 Lux. This study was conducted after review and approval by the Medytox Animal Experiment Ethics Committee.

As a control group, 100 units of Coretox (botulinum toxin type A, Lot No.: NSA20015, Medytox) was used. Recombinant botulinum toxin pBT145 was prepared at 1939 U/mL and used in the experiment. The potency of recombinant botulinum toxin was tested using a mouse potency test method based on the potency value derived from measurement before the potency test. For the placebo (placebo administration group), except for the botulinum toxin, the remaining excipient components were used the same as the recombinant botulinum toxin. In the potency test, the administered dose of each test substance was administered equally based on unit (U: a value representing the biological activity of botulinum toxin, where 1 U is the half-lethal dose of mice administered by intraperitoneal injection).

Coretox was diluted using sterilized saline solution (35V3AF3, Daehan Pharmaceutical Industry), and recombinant botulinum toxin pBT145 was diluted using placebo to concentrations of 6, 20, 60, and 200 U/mL, respectively. The day the test substance was administered was set as day 0, after anesthetizing the mice using an injection anesthetic (100 mg/kg ketamine hydrochloride + 10 mg/kg xylazine), each test substance was administered at 0.2 mL/kg to the mice's right gastrocnemius muscle using a Hamilton syringe according to the composition of the group in Table 3.

**[Table 3]**

| Group | Number of animals | Administered substance | Dose (U/kg) | Administration route | Evaluation indicator |
|---|---|---|---|---|---|
| 1 | 6 | Placebo | 0 | Right gastrocnemius muscle | DAS, CMAP |
| 2 | 6 | Coretox | 1.2 | Right gastrocnemius muscle | DAS |
| 3 | 6 | Coretox | 4 | Right gastrocnemius muscle | DAS |
| 4 | 6 | Coretox | 12 | Right gastrocnemius muscle | DAS, CMAP |
| 5 | 6 | Coretox | 40 | Right gastrocnemius muscle | DAS |
| 6 | 6 | pBT145 | 1.2 | Right gastrocnemius muscle | DAS |
| 7 | 6 | pBT145 | 4 | Right gastrocnemius muscle | DAS |
| 8 | 6 | pBT145 | 12 | Right gastrocnemius muscle | DAS, CMAP |
| 9 | 6 | pBT145 | 40 | Right gastrocnemius muscle | DAS |

For evaluation, the digit abduction score (DAS) evaluation method, which visually evaluates the degree of muscle paralysis in mice, and the compound muscle action potential (CMAP) test method, which measures the action potential of muscles responding to external electrical stimulation, were used. The DAS value shown in Table 4 indicate the degree of muscle paralysis according to the form of the toe on the administered side in mice and the CMAP value indicate the degree of inhibition of direct muscle contraction caused by nerve block.

**[Table 4]**

| Score | Criteria |
|---|---|
| 0 | Normal, no difference from the shape of the foot on the non-injected side |
| 1 | The space between the toes is narrowed, or two toes are close together and the rest are fully extended |
| 2 | All toes are significantly narrowed, or all three toes are stuck together |
| 3 | The feet are curved and the four toes are stuck together. |
| 4 | Feet are curved and all toes are stuck together |

CMAP was measured in the right gastrocnemius muscle area of each mice using a Nicolet Viking Quest (Viasys Healthcare, Inc.) equipment. The measurement method is the same as Test Example 3 except for the stimulation level and period of 7 mA to 8 mA and 0.1 ms. According to the test group composition in Table 3, DAS measurements were evaluated from day 1 to day 77 after administration at the administered doses of 1.2, 4, 12, and 40 U/kg, and CMAP was measured from the date of administration to day 98 in subjects administered at a dose of 12 U/kg, and statistical processing was performed in the same manner as in Test Example 4.

After administering a single dose of Coretox and recombinant botulinum toxin pBT145 at doses of 1.2, 4, 12 and 40 U/kg to the right gastrocnemius muscle of a rat, DAS evaluation results showed that all subjects in the Coretox 1.2 U/kg administration group recovered on day 14, and in the group administered with the same dose of pBT145, all subjects recovered on day 21, an increase of 7 days. In addition, in the Coretox 4 U/kg administration group, all subjects recovered on day 28 after administration, but in the group administered with the same dose of pBT145, all subjects recovered on day 35, an increase of 7 days. In the Coretox 12 U/kg administration group, all subjects recovered on day 49 after administration, but in the group administered with the same dose of pBT145, all subjects recovered on day 56, an increase of 7 days. In addition, in the Coretox 40 U/kg administration group, all subjects recovered on day 70 after administration, but in the group administered with the same dose of pBT145, all subjects recovered on day 77, an increase of 7 days (FIG. 8A), and through comparison of DAS ED₅₀, it was confirmed that pBT145 showed approximately 1.2 times stronger potency than Coretox (FIG. 8B).

In the CMAP test, the pBT145 12 U/kg administration group showed significantly lower CMAP values compared to the Coretox 12 U/kg administration group, and in the Coretox 12 U/kg administration group, CMAP recovery was observed on day 84 after administration, and in the pBT145 12 U/kg administration group CMAP recovery was observed on day 98, an increase of 14 days (FIG. 8C). From the above results conducted in mice, the potency and duration of action of recombinant botulinum toxin pBT145 increased compared to Coretox.

### Mode for Invention

Unless otherwise defined, technical and scientific terms used herein have the same meanings as those commonly used in the field to which this disclosure belongs. For purposes of understanding this disclosure, the following definitions will apply and terms used in the singular include the plural and vice versa.

As used herein, the terms 'protein' and 'polypeptide' are used interchangeably herein to refer to polymers of amino acid residues and variants and synthetic analogs thereof. These terms apply to naturally occurring amino acid polymers, including non-naturally occurring amino acids in which one or more amino acid residues have been synthesized, for example chemical analogs of the related naturally occurring amino acids. These terms also include post-translational modifications of polypeptides, for example glycosylation, phosphorylation, and acetylation.

The term "botulinum toxin (BoNT)" encompasses any polypeptide or fragment of botulinum toxin. In embodiments, botulinum toxin refers to full-length botulinum toxin or botulinum toxin derived fragments. In embodiments, botulinum toxin may enter a neuron and execute an overall cellular mechanism to inhibit neurotransmitter release.

The term 'domain' refers to a given protein sequence and its conserved portions of tertiary structure that may evolve, function and exist independently of the rest of the protein chain. Because domains are independently stable, domains may be domain swapped by genetic engineering between one protein and another to create a chimera protein.

The term "percent identity" refers to the degree of amino acid sequence identity between polypeptides. For example, if the first amino acid sequence is identical to the second amino acid sequence, the first and second amino acid sequences exhibit 100% identity. The percent identity of two amino acid sequences may be determined using the algorithm of Karlin and Altschul Proc. Natl. Acad. Sci. USA 87:2264-68, 1990, modified from Karlin and Altschul Proc. Natl. Acad. Sci. USA 90:5873-77, 1993. Such algorithms are included in the Altschul, et al. J. Mol. Biol. 215:403-10, 1990 NBLAST and XBLAST programs (version 2.0). BLAST protein search is performed with the XBLAST program, score=50, wordlength=3 to obtain amino acid sequences homologous to the protein molecule of interest. If there is a gap between the two sequences, may be used as described in Gapped BLAST Altschul et al., Nucleic Acids Res. 25(17):3389-3402, 1997. When using BLAST and Gapped BLAST programs, default variables for each program (for example, XBLAST and NBLAST) may be used.

The term 'recombination' refers to the process in which elements that make up genes, such as Deoxyribonucleic Acid (DNA) or Ribonucleic Acid (RNA), are changed from their original sequence during disassembly and reassembly. Through molecular biology experiments, fragments of DNA may be artificially recombined. DNA that has been artificially recombined in this way is called recombinant DNA.

The term "variant" encompasses any variation in a nucleic acid or amino acid sequence, including, for example, nucleic acid or amino acid truncations, additions, deletions, substitutions, and any combination thereof. In other words, 'variants of the recombinant botulinum toxin type A light chain domain' may encompass truncation, addition, deletion, substitution, and any combination thereof of nucleic acids or amino acids consisting the recombinant botulinum neurotoxin type A light chain domain.

The term "substitution" may include any amino acid other than a wild-type residue normally found at any sequence position. These substitutions may be with non-polar (hydrophobic) amino acids for example glycine, alanine, valine, leucine, isoleucine, methionine, phenylalanine, tryptophan, and proline. Substitutions may be with polar (hydrophilic) amino acids for example, serine, threonine, cysteine, tyrosine, asparagine, and glutamine. Substitutions may be with electrically charged amino acids, for example negatively charged amino acids for example aspartic acid and glutamic acid and positively charged amino acids for example such as lysine, arginine, and histidine.

The term 'wild-type' refers to the phenotype of a common species occurring in nature. In general, the wild-type may be the product of a standard normal allele at a locus, as opposed to being generated by a non-standard mutant allele.

The term "subject" may be male or female. The subject may be a fully developed subject (for example, an adult) or an individual undergoing development (for example, a child, infant, or fetus). The subject may be a mammal. The mammal may be, but is not limited to, a human, non-human primate, mouse, rat, dog, cat, horse, or cow.

Most of the botulinum toxin preparations currently on the market are preparations whose active component is botulinum toxin type A1, and in the case of Myobloc, botulinum toxin type B1 is used as the active component. The duration of potency of botulinum toxin type A1 preparation is on average about 3 months, and in the case of botulinum toxin type B1 preparation, it is known to be shorter. Due to the nature of botulinum toxin preparations that must be administered by intramuscular injection, it is necessary to develop products with longer lasting properties than commercially available preparations in terms of patient convenience.

The inventors of the present disclosure divided the sequences of botulinum toxin type A1 and A4 light chains into first, second, third, and fourth domains, into a total of 4 domains as shown in FIG. 1, according to the domain characteristics in FIG. 2.

The first domain of the botulinum toxin type A1 light chain (SEQ ID NO: 1) and the first domain of the A4 light chain (SEQ ID NO: 5) are regions that mainly interconnect with the substrate SNAP25 and include an α-exosite. The first domain of the botulinum toxin type A1 and A4 light chains may be the amino acid at position 1 to the amino acid at positions 90 to 150 (for example, amino acids at positions 90, 95, 100, 105, 110, 115, 120, 125, 130, 135, 140, 145, and 150).

The second domain of the botulinum toxin type A1 light chain (SEQ ID NO: 2) and the second domain of the A4 light chain (SEQ ID NO: 6) are regions mainly involved in the enzymatic activity of the light chain and include 170 loops and is the region that binds to zinc, a cofactor. The second domain of botulinum toxin type A1 and A4 light chains may be amino acids at positions 91 to 151 (for example, amino acids at positions 91, 96, 101, 106, 111, 116, 121, 126, 131, 136, 141, 146, and 151) to an amino acid at position 210 to 270 (for example, an amino acid at position 210, 215, 220, 225, 230, 235, 240, 245, 250, 255, 260, 265, and 270).

The third domain of the botulinum toxin type A1 light chain (SEQ ID NO: 3) and the third domain of the A4 light chain (SEQ ID NO: 7) include loop 250 and loop 370 that stabilize substrate binding and is a region involved in both the α-exosite and β-exosite substrate binding sites. The third domain of botulinum toxin type A1 and A4 light chains may be amino acids 211 to 271 (for example, amino acids 211, 216, 221, 226, 231, 236, 241, 246, 251, 256, 261, 266, and 271) to amino acids 386 to 446 (for example, amino acids 386, 391, 396, 401, 406, 411, 416, 421, 426, 431, 436, 441, and 446).

The fourth domain of the botulinum toxin type A1 light chain (SEQ ID NO: 4) and the fourth domain of the A4 light chain (SEQ ID NO: 8) are regions of the botulinum toxin type A light chain that are involved in structural flexibility. The fourth domain of the botulinum toxin type A1 and A4 light chains may be amino acids 362 to 425 (for example, amino acids 362, 367, 372, 377, 382, 387, 392, 397, 402, 407, 412, 417, 422, and 425) to amino acids 410 to 448 (for example, amino acids 410, 414, 417, 420, 425, 430, 435, 440, 445, and 448).

According to an aspect of the present disclosure, provided is a recombinant botulinum toxin type A light chain in which the sequence of the second domain of the first, second, third, and fourth domains of a botulinum toxin type A light chain other than botulinum toxin type A1 is substituted with a sequence of the second domain of botulinum toxin type A1 light chain or a variant thereof.

In an embodiment, botulinum toxin type A light chain other than botulinum toxin type A1 may be a botulinum toxin type A2 light chain (SEQ ID NO: 10), or a botulinum toxin type A3 light chain (SEQ ID NO: 11), a botulinum toxin type A4 light chain (SEQ ID NO: 12), a botulinum toxin type A5 light chain (SEQ ID NO: 13), botulinum toxin type A6 light chain (SEQ ID NO: 14), botulinum toxin type A7 light chain (SEQ ID NO: 15), or botulinum toxin type A8 light chain (SEQ ID NO: 16). For example, the botulinum toxin type A light chain may be a botulinum toxin type A4 light chain.

In an embodiment, the second domain of the botulinum toxin type A1 light chain may have the sequence of SEQ ID NO: 2. In another embodiment, the variant of the second domain of the botulinum toxin type A1 light chain is the same as the second domain of the botulinum toxin type A1 light chain, which is mainly involved in the enzymatic activity of the light chain, includes 170 loops, and binds to zinc, a cofactor, and includes an amino acid sequence with at least 90%, at least 91%, at least 92%, at least 93%, at least 94%, at least 95%, at least 96%, at least 97%, at least 98%, at least 99%, or at least 99.5% identity to SEQ ID NO: 2.

According to another aspect of the present disclosure, provided is a recombinant botulinum toxin type A light chain wherein the sequence of the fourth domain of the first, second, third, and fourth domains of the recombinant botulinum toxin type A light chain is further substituted with a sequence of the fourth domain of botulinum toxin type A1 light chain or a variant thereof.

In an embodiment, the fourth domain of the botulinum toxin type A1 light chain may be SEQ ID NO: 4. In another embodiment, a variant of the fourth domain of the botulinum toxin type A1 light chain is involved in the structural flexibility of the botulinum toxin type A light chain in the same manner as the botulinum toxin type A1 light chain fourth domain and includes an amino acid sequence with at least 90%, at least 91%, at least 92%, at least 93%, at least 94%, at least 95%, at least 96%, at least 97%, at least 98%, at least 99%, or at least 99.5% identity to SEQ ID NO: 4.

According to another aspect of the present disclosure provided is a recombinant botulinum toxin including the recombinant botulinum toxin type A light chain, and a botulinum toxin heavy chain.

In an embodiment, the botulinum toxin heavy chain may be a botulinum toxin type A heavy chain, a botulinum toxin type B heavy chain, a botulinum toxin type C heavy chain, a botulinum toxin type D heavy chain, a botulinum toxin type E heavy chain, a botulinum toxin type F heavy chain, or a botulinum toxin type G heavy chain.

In an embodiment, the botulinum toxin heavy chain may be a botulinum toxin type A heavy chain. For example, the botulinum toxin heavy chain may be a botulinum toxin type A1 heavy chain, a botulinum toxin type A2 heavy chain, a botulinum toxin type A3 heavy chain, a botulinum toxin type A4 heavy chain, a botulinum toxin type A5 heavy chain, a botulinum toxin type A6 heavy chain, a botulinum toxin type A7 heavy chain, or a botulinum toxin type A8 heavy chain. For example, the botulinum toxin heavy chain may be a botulinum toxin type A1 heavy chain (SEQ ID NO: 17).

In an embodiment, the recombinant botulinum toxin may be a recombinant botulinum toxin in which a recombinant botulinum toxin type A light chain and a botulinum toxin heavy chain are linked by a disulfide bond. For example, recombinant botulinum toxin is produced in clostridium bacteria as a single, relatively inactive polypeptide chain weighing about 150 kDa with a high degree of amino acid sequence identity between botulinum toxin types. The single polypeptide is then cleaved into an approximately 100 kDa heavy chain and a 50 kDa light chain, and the heavy chain and light chain may form a disulfide bond.

According to another aspect of the present disclosure, provided is a composition including the recombinant botulinum toxin, and a pharmaceutically acceptable excipient or additive.

In an embodiment, the pharmaceutically acceptable excipient or additive may be a stabilizer, an ionic compound, a surfactant, a buffer, a lyophilization protectant, or a combination thereof, for example an amino acid (for example, methionine), salt (for example, NaCl), buffer solution, non-ionic surfactant (for example, polysorbate, for example, polysorbate 20), sugar (for example, disaccharide, such as white sugar), sugar alcohol (for example, sorbitol), or a combination thereof.

In an embodiment, the composition may not include albumin or animal-derived components. For example, compositions that do not include albumin or animal-derived components include those disclosed in WO2009-008595A1 or WO2012-134240A2, the contents of which are incorporated herein by reference in their entirety.

In an embodiment, the composition may be formulated in any form, such as a solid or liquid preparation, for example, a lyophilized powder, a liquid, or a pre-filled syringe preparation.

According to another aspect of the present disclosure, provided is a use of a composition including the recombinant botulinum toxin, and a pharmaceutically acceptable excipient or additive, for administration to a subject to ameliorate or treat a disease.

In an embodiment, the composition including the recombinant botulinum toxin and a pharmaceutically acceptable excipient or additive may be administered to a subject to ameliorate wrinkles, square jaw, pointed chin, wounds, skin softening, scars, acne, pores, elasticity, or keloids.

In an embodiment, the composition including the recombinant botulinum toxin and a pharmaceutically acceptable excipient or additive may be administered to a subject to treat facial spasm, eyelid spasm, torticollis, blepharospasm, cervical dystonia, mid-pharyngeal dystonia, spasmodic dysphonia, migraine, anal pruritus, or hyperhidrosis.

In an embodiment, the composition may be administered by transdermal, subcutaneous, or intramuscular administration. In an embodiment, this may be accomplished by topically administering the composition to a muscle or group of muscles. In an embodiment, the reduction of forehead wrinkles and skin wrinkles may be achieved by administering the composition transdermally or subcutaneously to the wrinkles.

According to another aspect of the present disclosure, provided is a method of ameliorating or treating a disease by administering to a subject a composition further including the recombinant botulinum toxin and a pharmaceutically acceptable excipient or additive.

In an embodiment, the method may be for ameliorating wrinkles, square jaw, pointed chin, wounds, skin softening, scars, acne, pores, elasticity, or keloid symptoms, the method including administering the composition to a subject.

In an embodiment, the method may be for treating facial spasm, eyelid spasm, torticollis, blepharospasm, cervical dystonia, mid-pharyngeal dystonia, spasmodic dysphonia, migraine, anal pruritus, or hyperhidrosis, the method including administering the composition to a subject.

According to another aspect of the present disclosure, provided is a method of preparing a recombinant botulinum toxin type A light chain with an increased potency or half-life compared to a wild-type botulinum toxin type A1 light chain, the method including substituting a sequence of the second domain of the first, second, third, and fourth domains of a botulinum toxin type A light chain other than botulinum toxin type A1 with a sequence of the second domain of botulinum toxin type A1 or a variant thereof.

In an embodiment, the method may be for preparing a recombinant botulinum toxin type A light chain with an increased potency or half-life compared to a wild-type botulinum toxin type A1 light chain may include substituting a sequence of the second domain of the botulinum toxin type A4 light chain with a sequence of the second domain of botulinum toxin type A1 or a variant thereof.

For example, the method may be for producing a recombinant botulinum toxin type A light chain with increased potency or half-life compared to wild-type botulinum toxin, that includes substituting four domains of botulinum toxin type A1 light chain and botulinum toxin type A4 light chain genes with each other; cloning genes including a wild-type botulinum toxin type A light chain and a domain substituted recombinant botulinum toxin type A light chain, respectively, introducing them into *Escherichia coli* (*E. Coli*), followed by inducing expression of the domain substituted recombinant botulinum toxin type A light chain; producing a recombinant botulinum toxin A light chain (pBT114, SEQ ID NO: 30) in which the sequence of the second domain of the botulinum toxin type A4 light chain is substituted with the sequence of the second domain of botulinum toxin type A1 or a variant thereof.

According to another aspect of the present disclosure, provided is a method of preparing a recombinant botulinum toxin type A light chain with an increased potency or half-life compared to a wild-type botulinum toxin type A1, the method including additionally substituting a sequence of the fourth domain of the first, second, third, and fourth domains of the recombinant botulinum toxin type A light chain with a sequence of a fourth domain of botulinum toxin type A4 or a variant thereof.

In an embodiment, the method may be for preparing a recombinant botulinum toxin type A light chain with an increased potency or half-life compared to a wild-type botulinum toxin type A1 may include additionally substituting a sequence of the fourth domain of the recombinant botulinum toxin type A light chain with a sequence of the fourth domain of botulinum toxin type A1 or a variant thereof.

For example, the method may be for producing a recombinant botulinum toxin type A light chain with increased potency or half-life compared to wild-type botulinum toxin type A1, that includes substituting four domains of botulinum toxin type A1 light chain and botulinum toxin type A4 light chain genes with each other; cloning genes including a wild-type botulinum toxin type A light chain and a domain substituted recombinant botulinum toxin type A light chain, respectively, introducing them into *Escherichia coli* (*E. Coli*), followed by inducing expression of the domain substituted recombinant botulinum toxin type A light chain; producing a recombinant botulinum toxin A light chain (pBT115, SEQ ID NO: 31) in which the sequence of the second domain and the fourth domain of the botulinum toxin type A4 light chain is substituted with the sequence of the second domain, fourth domain of botulinum toxin type A1 or a variant thereof.

Another aspect of the present disclosure provides a cell including a nucleic acid including a polynucleotide encoding the recombinant botulinum toxin type A light chain and a polynucleotide encoding the botulinum toxin heavy chain, or a vector including the same. The cells may express a recombinant botulinum toxin including the recombinant botulinum toxin type A light chain, and the botulinum toxin heavy chain.

According to another aspect of the present disclosure, provided is a method of producing a recombinant botulinum toxin with increased potency or half-life compared to a wild-type botulinum toxin including culturing cells including nucleic acid or vector including the same including a polynucleotide encoding a recombinant botulinum toxin type A light chain, and a polynucleotide encoding a botulinum toxin heavy chain. The method may further include isolating the recombinant botulinum toxin from the culture.

In an embodiment, the botulinum toxin heavy chain may be a botulinum toxin type A1 heavy chain, botulinum toxin type A2 heavy chain, botulinum toxin type A3 heavy chain, botulinum toxin type A4 heavy chain, botulinum toxin type A5 heavy chain, botulinum toxin type A6 heavy chain, botulinum toxin type A7 heavy chain, or botulinum toxin type A8 heavy chain. In an embodiment, the botulinum toxin heavy chain may be a botulinum toxin type A1 heavy chain (SEQ ID NO: 17).

For example, the method may be a method of producing a recombinant botulinum toxin with increased potency or half-life compared to a wild-type botulinum toxin, the method including cloning a full-length recombinant botulinum toxin gene (pBT145, pBT146) including a domain substituted recombinant botulinum toxin type A light chain (pBT114, pBT115) and a botulinum toxin type A1 heavy chain; introducing the cloned toxin gene into an inactivated detoxified strain and culturing the inactivated detoxified strain into which the toxin gene was introduced [Bradshaw et al. 2010, Pellett et al. 2016] to produce a botulinum toxin with a substituted domain; and purifying and isolating a 150 kDa form of recombinant toxin without complex components from the culture.

## Claims

1. A recombinant botulinum toxin type A light chain, wherein a sequence of a second domain of first, second, third, and fourth domains of a botulinum toxin type A light chain, other than botulinum toxin type A1, is substituted with a sequence of a second domain of botulinum toxin type A1 or a variant thereof.

2. The recombinant botulinum toxin type A light chain of claim 1, wherein a sequence of the fourth domain of the first, second, third, and fourth domains of the recombinant botulinum toxin type A light chain is further substituted with a sequence of a fourth domain of botulinum toxin type A1 or a variant thereof.

3. The recombinant botulinum toxin type A light chain of claim 1 or claim 2, wherein the botulinum toxin type A light chain is botulinum toxin type A2, A3, A4, A5, A6, A7 or A8.

4. The recombinant botulinum toxin type A light chain of claim 3, wherein the botulinum toxin type A light chain is botulinum toxin type A4.

5. A recombinant botulinum toxin comprising the recombinant botulinum toxin type A light chain of any one of claim 1 to claim 4, and a botulinum toxin heavy chain.

6. The recombinant botulinum toxin of claim 5, wherein the botulinum toxin type A heavy chain is botulinum toxin type A, B, C, D, E, F, or G.

7. The recombinant botulinum toxin of claim 6, wherein the botulinum toxin type A heavy chain is botulinum toxin type A1, A2, A3, A4, A5, A6, A7, or A8.

8. The recombinant botulinum toxin of claim 7, wherein the botulinum toxin type A heavy chain is botulinum toxin type A1.

9. A composition comprising the recombinant botulinum toxin type A of any one of claim 5 to claim 8, and a pharmaceutically acceptable excipient or additive.

10. The composition of claim 9, wherein the pharmaceutically acceptable excipient or additive is a stabilizer, an ionic compound, a surfactant, a buffer, a lyophilization protectant, or a combination thereof.

11. The composition of claim 10, wherein the pharmaceutically acceptable excipient or additive is an amino acid, a salt, a buffer solution, a non-ionic surfactant, a sugar, a sugar alcohol, or a combination thereof.

12. The composition of any one of claim 9 to claim 11, wherein the composition does not comprise albumin or an animal-derived component.

13. The composition of any one of claim 9 to claim 12, in the form of a lyophilized powder, liquid, or prefilled syringe preparation.

14. The composition of any one of claim 9 to claim 13, wherein the composition is for ameliorating wrinkles, square jaw, pointed chin, wounds, skin softening, scars, acne, pores, elasticity, or keloid symptoms.

15. The composition of any one of claim 9 to claim 13, wherein the composition is for treating facial spasm, eyelid spasm, torticollis, blepharospasm, cervical dystonia, mid-pharyngeal dystonia, spasmodic dysphonia, migraine, anal pruritus, or hyperhidrosis.

16. The composition of any one of claim 9 to claim 15, wherein the composition is for transdermal, subcutaneous, or intramuscular administration.

17. A use of the composition according to any one of claim 10 to claim 14 for ameliorating wrinkles, square jaw, pointed chin, wounds, skin softening, scars, acne, pores, elasticity, or keloid symptoms.

18. The use of the composition according to any one of claim 10 to claim 14 for treating facial spasm, eyelid spasm, torticollis, blepharospasm, cervical dystonia, mid-pharyngeal dystonia, spasmodic dysphonia, migraine, anal pruritus, or hyperhidrosis.

19. A method of ameliorating wrinkles, square jaw, pointed chin, wounds, skin softening, scars, acne, pores, elasticity, or keloids, the method comprising administering the composition of any one of claim 9 to claim 13 to a subject.

20. A method of treating facial spasm, eyelid spasm, torticollis, blepharospasm, cervical dystonia, mid-pharyngeal dystonia, spasmodic dysphonia, migraine, anal pruritus, or hyperhidrosis, the method comprising administering the composition of any one of claim 9 to claim 13 to a subject.

21. A method of preparing a recombinant botulinum toxin type A light chain with an increased potency or half-life compared to a wild-type botulinum toxin type A light chain, the method comprising substituting a sequence of a second domain of first, second, third, and fourth domains of a botulinum toxin type A light chain, other than botulinum toxin type A1, with a sequence of a second domain of botulinum toxin type A1 or a variant thereof.

22. The method of claim 21, comprising additionally substituting a sequence of the fourth domain of the first, second, third, and fourth domains of the recombinant botulinum toxin type A light chain with a sequence of a fourth domain of botulinum toxin type A1 or a variant thereof.

23. The method of claim 21 or claim 22, wherein the botulinum toxin type A light chain is botulinum toxin type A1, A2, A3, A5, A6, A7, or A8.

24. The method of claim 23, wherein the botulinum toxin type A light chain is botulinum toxin type A4.

25. A method of preparing a recombinant botulinum toxin with an increased potency or half-life compared to a wild-type botulinum toxin, the method comprising culturing a cell that comprises a nucleic acid molecule or a vector comprising the same, the nucleic acid molecule comprising a polynucleotide encoding the recombinant botulinum toxin type A light chain of any one of claim 1 to claim 4 and a polynucleotide encoding a botulinum toxin heavy chain.

26. The method of claim 25, wherein the botulinum toxin heavy chain is botulinum toxin type A, B, C, D, E, F, or G.

27. The method of claim 26, wherein the botulinum toxin heavy chain is botulinum toxin type A1, A2, A3, A4, A5, A6, A7, or A8.

28. The method of claim 27, wherein the botulinum toxin heavy chain is botulinum toxin type A1.
